(19) Europäisches Patentamt
European Patent Office
Office européen des brevets

(11) Veröffentlichungsnummer: **0 080 071**
**B1**

(12) # EUROPÄISCHE PATENTSCHRIFT

(45) Veröffentlichungstag der Patentschrift: **13.05.87**

(51) Int. Cl.⁴: **C 07 D 405/06**

(21) Anmeldenummer: **82109961.1**

(22) Anmeldetag: **28.10.82**

(54) **2-Imidazolylmethyl-2-phenyl-1,3-dioxolane, Verfahren zu ihrer Herstellung sowie ihre Verwendung als Fungizide.**

(30) Priorität: **07.11.81 DE 3144318**

(43) Veröffentlichungstag der Anmeldung: **01.06.83 Patentblatt 83/22**

(45) Bekanntmachung des Hinweises auf die Patenterteilung: **13.05.87 Patentblatt 87/20**

(84) Benannte Vertragsstaaten: **AT BE CH DE FR GB IT LI NL SE**

(56) Entgegenhaltungen:
**EP - A - 0 029 355**
**EP - A - 0 065 485**
**GB - A - 2 026 486**
**NL - A - 7 514 381**
**US - A - 3 575 999**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Knops, Hans-Joachim, Dr., Claudiusweg 3, D-5600 Wuppertal 1 (DE)**
Erfinder: **Büchel, Karl Heinz, Prof. Dr., Dabringhausener Strasse 42, D-5093 Burscheid (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen (DE)**

**EP 0 080 071 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-Imidazolylmethyl-2-phenyl-1,3-dioxolane, ein Verfahren zu ihrer Herstellung sowie ihre Verwendung als Fungizide.

Es ist bereits bekannt geworden, dass bestimmte Triazolylalkanole fungizide Eigenschaften aufweisen (vgl. DE-A-2 431 407). So lässt sich zum Beispiel

4-Chlorphenyl-(1,2,4-triazol-1-yl-methyl)-carbinol

zur Bekämpfung von Pilzen verwenden. Die Wirkung dieses Stoffes ist jedoch insbesondere bei niedrigen Aufwandmengen nicht immer ausreichend.

Weiterhin ist bereits bekannt geworden, dass bestimmte Imidazolyl-alkanole gute Wirkungen gegen humanpathogene Pilze besitzen (vgl. 7. Med. Chem. 12, 784–791 (1969)). So können zum Beispiel

4-Bromphenyl-(imidazol-1-yl-methyl)-carbinol

und

2,4-Dichlorphenyl-(imidazol-1-yl-methyl)-carbinol

für diese Indikation eingesetzt werden. Die Wirkung dieser Verbindungen gegen phytopathogene Pilze ist jedoch nicht immer befriedigend.

Ferner sind zahlreiche 2-Imidazolylmethyl-2-phenyl-1,3-dioxolane mit fungiziden Eigenschaften bekannt (vgl. EP-A-0 029 355, GB-A-2 026 486, US-A-3 575 999 und NL-A-7 514 381). Es wurden jedoch keine Verbindungen dieses Typs offenbart, in denen die Phenyl-Gruppe durch Trifluormethyl und gegebenenfalls einen weiteren Rest substituiert ist, oder in denen die Phenyl-Gruppe mit einem weiteren substituierten Phenylrest verbunden ist.

Schliesslich werden in der vorgängigen, aber nicht vorveröffentlichten Anmeldung gemäss EP-A-0 065 485 unter anderem mikrobizid wirksame 2-Imidazolyl-methyl-2-phenyl-1,3-dioxolane beschrieben, die dadurch charakterisiert sind, dass der Phenyl-Rest zwingend noch eine gegebenenfalls substituierte Phenoxy- oder Naphthoxy-Gruppe trägt. Entsprechende Verbindungen, in denen diese gegenbenenfalls substituierte Phenoxy- oder Naphthoxy-Gruppe fehlt, werden nicht erwähnt.

Es wurden nun neue Imidazolylmethyl-2-phenyl-1,3-dioxolane der Formel

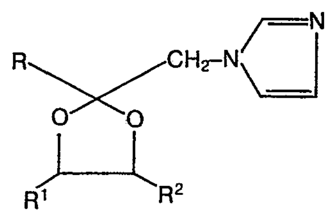

(I)

in welcher

R für die Gruppierungen

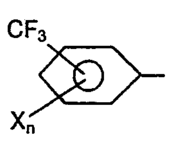 und 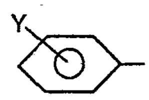 steht,

$R^1$ für Wasserstoff oder Alkyl mit 1 bis 4 Kohlenstoffatomen steht;

$R^2$ für Wasserstoff, Alkyl mit 1 bis 4 Kohlenstoffatomen oder Halogenalkyl mit 1 bis 2 Kohlenstoffatomen und 1 bis 5 gleichen oder verschiedenen Halogenatomen steht;

X für Wasserstoff, Fluor, Chlor, Brom und Alkyl oder Alkoxy mit jeweils 1 bis 4 Kohlenstoffatomen steht;

n für 1 steht und

Y für substituiertes Phenyl steht, wobei als Substituenten infrage kommen: Halogen oder Alkyl mit 1 bis 4 Kohlenstoffatomen,

sowie deren pflanzenverträgliche Säureadditions-Salze und Metallsalz-Komplexe gefunden.

Die Verbindungen der Formel (I) können in verschiedenen stereoisomeren Formen vorkommen; vorzugsweise fallen sie in Form von Stereoisomerengemischen an.

Weiterhin wurde gefunden, dass man die 2-Imidazolyl-2-phenyl-1,3-dioxolane der Formel (I) sowie deren pflanzenverträgliche Säureadditions-Salze und Metallsalz-Komplexe erhält, wenn man 1,3-Dioxolan-Derivate der Formel

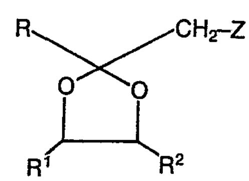

(II)

in welcher

R, $R^1$ und $R^2$ die oben angegebene Bedeutung haben und

Z für Halogen, sowie die Gruppierung $-O-SO_2-R^3$ steht, wobei

$R^3$ für Methyl oder p-Methylphenyl steht,

mit Imidazolen der Formel

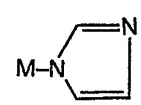

(III)

in welcher

M für Wasserstoff oder ein Alkalimetall steht,

in Gegenwart eines Verdünnungsmittels und gegebenenfalls in Gegenwart eines Säurebindemittels umsetzt, und gegebenenfalls anschliessend an die so erhaltenen Verbindungen der Formel (I) eine Säure oder ein Metallsalz addiert.

Schliesslich wurde gefunden, dass die erfindungsgemässen 2-Imidazolyl-2-phenyl-1,3-dioxolane der Formel (I) sowie deren pflanzenverträgliche Säureadditions-Salze und Metallsalz-Komplexe starke fungizide Eigenschaften aufweisen.

Überraschenderweise zeigen die erfindungsgemässen Stoffe eine bessere fungizide Wirksamkeit als die aus dem Stand der Technik bekannten Verbindungen

4-Chlorphenyl-(1,2,4-triazol-1-yl-methyl)-carbinol,

4-Bromphenyl-(imidazol-1-yl-methyl)-carbinol

und

2,4-Dichlorphenyl-(imidazol-1-yl-methyl)-carbinol,

welches konstitutionell ähnliche Stoffe gleicher Wirkungsrichtung sind.

Die erfindungsgemässen 2-Imidazolyl-2-phenyl-1,3-dioxolane sind durch die Formel (I) allgemein definiert. Sie unterscheiden sich von den Verbindungen, die aus der EP-A-0 029 355, der GB-A-2 026 486, der US-A-3 575 999, der NL-A-7 514 381 und der EP-A-0 065 485 bekannt sind, in charakteristischer Weise durch die Substituenten, die am Phenyl-Rest enthalten sind.

Bevorzugt sind diejenigen Verbindungen der Formel (I), in denen

R für die Gruppierungen

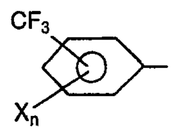 und 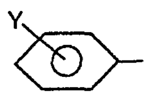 steht;

$R^1$ für Wasserstoff oder Alkyl mit 1 bis 4 Kohlenstoffatomen steht,

$R^2$ für Wasserstoff, Alkyl mit 1 bis 4 Kohlenstoffatomen und durch Fluor oder Chlor substituiertes Methyl steht;

X für Wasserstoff, Fluor, Chlor oder Methyl steht;

n für 1 steht und

Y für substituiertes Phenyl steht, wobei Fluor, Chlor und Methyl als Substituenten genannt seien.

Bevorzugte erfindungsgemässe Stoffe sind ausserdem Säureadditions-Salze von 2-Imidazolylmethyl-2-phenyl-1,3-dioxolanen der Formel (I), in denen R, $R^1$ und $R^2$ die vorzugsweise genannten Bedeutungen haben. Besonders bevorzugt sind dabei solche Säureadditions-Salze, die durch Addition von Halogenwasserstoffsäure, wie zum Beispiel Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäuren, wie zum Beispiel p-Toluolsulfonsäure und 1,5-Naphthalindisulfonsäure entstehen.

Bevorzugte erfindungsgemässe Stoffe sind auch Metallsalz-Komplexe von 2-Imidazolylmethyl-2-phenyl-1,3-dioxolanen der Formel (I), in denen R, $R^1$ und $R^2$ die vorzugsweise genannten Bedeutungen haben. Besonders bevorzugt sind dabei solche Metallsalz-Komplexe, die als Kationen Metalle der II. bis IV. Hauptgruppe oder der I. und II. oder IV. bis VIII. Nebengruppe des Periodensystems der Elemente enthalten, wobei Kupfer, Zink, Mangan, Magnesium, Zinn, Eisen und Nickel beispielhaft genannt seien.

Anionen dieser Metallsalz-Komplexe sind vorzugsweise solche, die sich von Halogenwasserstoffsäuren, insbesondere Chlorwasserstoffsäure und Bromwasserstoffsäure, ferner von Phosphorsäure, Salpetersäure und Schwefelsäure ableiten.

Verwendet man beispielsweise

2-Brommethyl-2-(4-chlor-2-trifluormethyl)-phenyl-1,3-dioxolan

und Imidazol als Ausgangsstoffe, so kann der Reaktionsablauf nach dem erfindungsgemässen Verfahren durch das folgende Formelschema wiedergegeben werden:

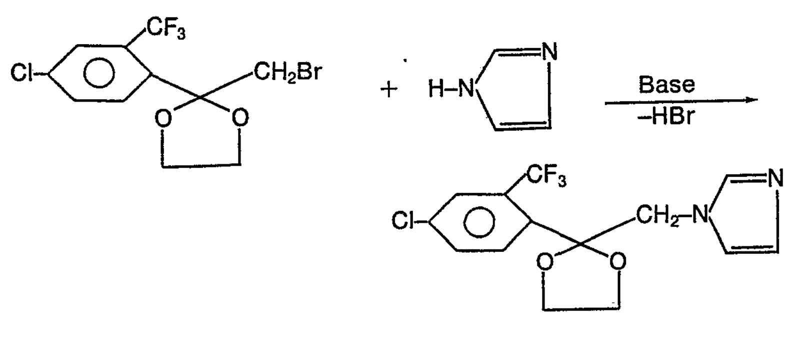


Die bei der Durchführung des erfindungsgemässen Verfahrens als Ausgangsstoffe benötigten 1,3-Dioxolan-Derivate sind durch die Formel (II) allgemein definiert. In dieser Formel stehen R, $R^1$ und $R^2$ für die Bedeutungen, die bei der Beschreibung der erfindungsgemässen Stoffe der Formel (I) bereits für diese Reste genannt wurden. Z steht vorzugsweise für Chlor, Brom oder die Gruppierung $-O-SO_2-R^3$, worin $R^3$ für Methyl oder p-Methylphenyl steht.

Die 1,3-Dioxolan-Derivate der Formel (II) sind noch nicht bekannt. Sie werden erhalten, indem man Keto-Derivate der Formel

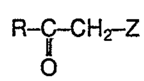 (IV)

in welcher

R und Z die oben angegebene Bedeutung haben,

mit Diolen der Formel

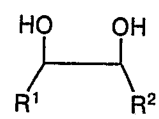 (V)

in welcher

$R^1$ und $R^2$ die oben angegebene Bedeutung haben,

in Gegenwart eines inerten organischen Lösungsmittels, wie beispielsweise Toluol, und in Gegenwart einer starken Säure als Katalysator, wie beispielsweise p-Toluolsulfonsäure, bei Temperaturen zwischen 80 und 100 °C, gegebenenfalls unter erhöhtem Druck, umsetzt.

Die Keto-Derivate der Formel (IV) sind bekannt (vgl. z.B. DE-A-2 431 407) oder werden in allgemein bekannter Art und Weise erhalten, indem man die entsprechenden Ketone z.B. mit Chlor oder Brom in Gegenwart eines inerten organischen Lösungsmittels, wie beispielsweise eines Ethers, eines chlorierten oder nicht chlorierten Kohlenwasserstoffes, bei Raumtemperatur umsetzt, oder mit üblichen Chlorierungsmitteln, wie beispielsweise Sulfurylchlorid, bei 20 bis 60°C umsetzt. Die Sulfonsäure-Derivate der Verbindungen der Formel (IV) lassen sich ebenfalls nach bekannten Methoden herstellen.

Die Diole der Formel (V) sind allgemein bekannte Verbindungen der organischen Chemie; sie werden in allgemein bekannter Art und Weise erhalten.

Die ausserdem für das erfindungsgemässe Verfahren als Ausgangsstoffe zu verwendenden Imidazole sind durch die Formel (III) allgemein definiert. In dieser Formel steht M vorzugsweise für Wasserstoff, Natrium und Kalium.

Die Imidazole der Formel (III) sind allgemein bekannte Verbindungen der organischen Chemie.

Als Verdünnungsmittel kommen für das erfindungsgemässe Verfahren inerte organische Lösungsmittel infrage. Hierzu gehören vorzugsweise Amide, wie Dimethylformamid oder Dimethylacetamid; ferner Dimethylsulfoxid oder Hexamethylphosphorsäuretriamid.

Das erfindungsgemässe Verfahren wird gegebenenfalls in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise
Triethylamin, N,N-Dimethylcyclohexylamin,
Dicyclohexylamin, N,N-Dimethylbenzylamin,
weiterhin Pyridin und Diazabicyclooctan. Vorzugsweise verwendet man einen entsprechenden Überschuss an Imidazol.

Die Reaktionstemperaturen können bei der Durchführung des erfindungsgemässen Verfahrens in einem grösseren Bereich variiert werden. Im allgemeinen arbeitet man bei Temperaturen zwischen 20 und 150°C, vorzugsweise zwischen 60 und 150°C.

Bei der Durchführung des erfindungsgemässen Verfahrens setzt man auf 1 Mol der Verbindungen der Formel (II) vorzugsweise 1 bis 2 Mol des Imidazols der Formel (III) und gegebenenfalls 1 bis 2 Mol Säurebinder ein. Die Isolierung der Verbindungen der Formel (I) erfolgt in üblicher Art und Weise. In manchen Fällen erweist es sich als vorteilhaft, die Verbindungen der Formel (I) über ihre Salze in reiner Form darzustellen.

Zur Herstellung von pflanzenverträglichen Säureadditions-Salzen der Verbindungen der Formel (I) kommen vorzugsweise diejenigen Säuren in Frage, die bereits im Zusammenhang mit der Beschreibung der erfindungsgemässen Säureadditions-Salze als bevorzugte Säuren genannt wurden.

Diese Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren isoliert und gegebenenfalls durch Waschen mit einem inerten organischen Lösungsmittel gereinigt werden.

Zur Herstellung von Metallsalz-Komplexen von Verbindungen der Formel (I) kommen vorzugsweise Salze von denjenigen Anionen und Kationen in Betracht, die bereits im Zusammenhang mit der Beschreibung der erfindungsgemässen Metallsalz-Komplexe als bevorzugt genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Podosphaera-Arten, wie gegen den Erreger des echten Apfelmehltaus (Podosphaera leucotricha) sowie zur Bekämpfung von Getreidekrankheiten, wie gegen den Erreger des echten Getreidemehltaus (Erysiphe graminis) und gegen die Streifenkrankheit der Gerste (Helminthosporium gramineum) verwendet werden. Die erfindungsgemässen Verbindungen zeigen auch eine gute in-vitro-Wirkung, insbesondere gegen Krankheitserreger an Reispflanzen.

In entsprechenden Aufwandmengen zeigen die erfindungsgemässen Verbindungen auch pflanzenwachstumsregulierende Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut,

ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemässen Wirkstoffe werden durch die nachfolgenden Beispiele veranschaulicht.

Herstellungsbeispiele

Beispiel 1

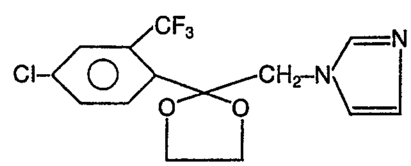

× HCl

3,9 g (0,0116 Mol)
2-Brommethyl-2-(4-chlor-2-trifluormethyl)-phenyl-1,3-dioxolan
und 2,4 g (0,0348 Mol) Imidazol werden in 50 ml Dimethylacetamid 4 Tage unter Rückfluss erhitzt. Nach dem Abkühlen wird das Reaktionsgemisch in Wasser gelöst und mit Ether extrahiert. Die Etherphase wird mit Wasser gewaschen, über Natriumsulfat getrocknet und eingeengt. Das zurückbleibende Öl wird mit etherischer Salzsäure versetzt und durch Anreiben zur Kristallisation gebracht. Man erhält
2-(4-Chlor-2-trifluormethyl)-phenyl-2-(imidazol-1-yl)-methyl-1,3-dioxolan-hydrochlorid
vom Schmelzpunkt 174–83 °C.

Herstellung des Ausgangsproduktes

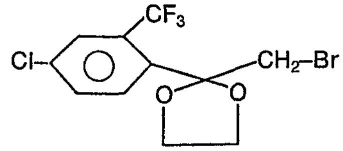

35 g (0,12 Mol) 4-Chlor-2-trifluormethyl-phenacylbromid und 9 g (0,15 Mol) Glykol werden mit 1,5 g p-Toluolsulfonsäure in 80 ml Toluol in Gegenwart von 20 ml Butanol 24 Stunden am Wasserabscheider erhitzt. Nach dem Abkühlen wird das Reaktionsgemisch nacheinander mit verdünnter wässriger Natronlauge und mit Wasser gewaschen, über Natriumsulfat getrocknet und eingeengt. Man erhält 29,6 g (74% der Theorie) rohes 2-Brommethyl-2-(4-chlor-2-trifluormethyl)-phenyl-1,3-dioxolan als Öl, das direkt weiter umgesetzt wird.

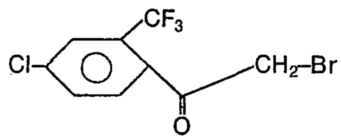

22,3 g (0,1 Mol) 4-Chlor-2-trifluormethyl-acetophenon in 200 ml Chloroform werden mit 1 ml Essigsäure versetzt und auf 40 °C erwärmt. Bei dieser Temperatur werden 5,1 ml (0,1 Mol) Brom in 50 ml Chloroform zugetropft. Die Reaktionsmischung wird bis zum Ende der Gasentwicklung gerührt. Nach dem Abkühlen wird nacheinander mit Wasser und wässriger Natriumhydrogencarbonatlösung gewaschen, über Natriumsulfat getrocknet und eingeengt. Man erhält 29,4 g (97% der Theorie) rohes 4-Chlor-2-trifluormethyl-phenacyl-bromid als Öl, das direkt weiter umgesetzt wird.

Nach der im Beispiel 1 angegebenen Methode werden auch die in der folgenden Tabelle 1 aufgeführten Verbindungen der Formel

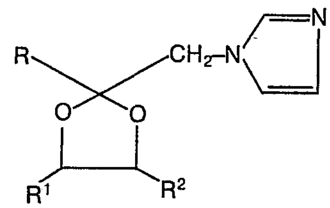 (I)

hergestellt:

Tabelle 1

| Bsp. Nr. | R | $R^1$ | $R^2$ | Physikal. Konstante |
|---|---|---|---|---|
| 2 | 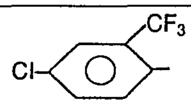 | $CH_3$ | H | zähes Oel |
| 3 | 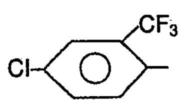 | $CH_2Cl$ | H | zähes Oel |
| 4 | 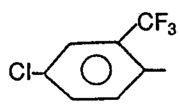 | $C_2H_5$ | H | zähes Oel |
| 5 | 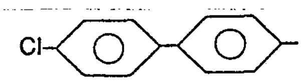 | H | H | Fp: 220 °C( × HCl) |
| 6 | 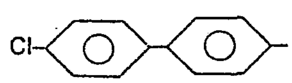 | $CH_3$ | H | zähes Oel |
| 7 | 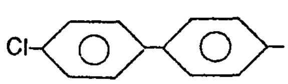 | $CH_2Cl$ | H | zähes Oel |
| 8 | 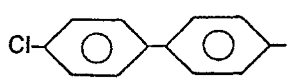 | $C_2H_5$ | H | zähes Oel |
| 9 | 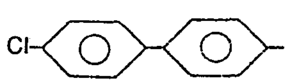 | $CH_3$ | $CH_3$ | zähes Oel |
| 10 | 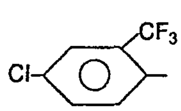 | $CH_3$ | $CH_3$ | zähes Oel |

Anwendungsbeispiele

In den folgenden Beispielen werden die nachstehend angegebenen Verbindungen als Vergleichssubstanzen eingesetzt:

(A) = 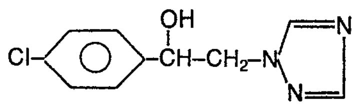

(B) = 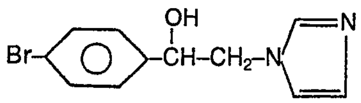

(C) = 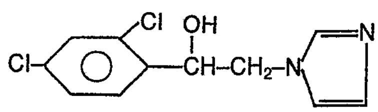

Beispiel A

Erysiphe-Test (Gerste) / protektiv

Lösungsmittel:

100 Gewichtsteile Dimethylformamid

Emulgator:

0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 2 und 4.

Beispiel B

Drechslera graminea-Test (Gerste) / Saatgutbehandlung (syn. Helminthosporium gramineum)

Die Anwendung der Wirkstoffe erfolgt als Trokkenbeizmittel. Sie werden zubereitet durch Abstrecken des jeweiligen Wirkstoffes mit Gesteinsmehl zu einer feinpulvrigen Mischung, die eine gleichmässige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschliessend sät man die vorgekeimte Gerste mit 2 × 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen bei diesem Test z.B. die Verbindungen gemäss folgender Herstellungsbeispiele: 1 und 2.

Beispiel C

Podosphaera-Test (Apfel) / protektiv

Lösungsmittel:

4,7 Gewichtsteile Aceton

Emulgator:

0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23 °C und einer relativen Luftfeuchtigkeit von ca. 70% aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 2, 4 und 1.

**Patentansprüche**

1. 2-Imidazolylmethyl-2-phenyl-1,3-dioxolane der Formel

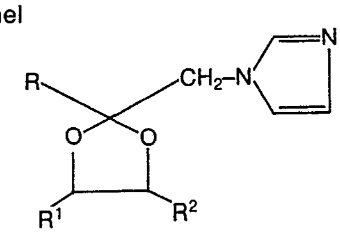 (I)

in welcher

R für die Gruppierungen

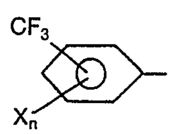 und 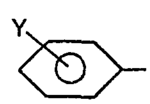 steht,

$R^1$ für Wasserstoff oder Alkyl mit 1 bis 4 Kohlenstoffatomen steht;

$R^2$ für Wasserstoff, Alkyl mit 1 bis 4 Kohlenstoffatomen oder Halogenalkyl mit 1 bis 2 Kohlenstoffatomen und 1 bis 5 gleichen oder verschiedenen Halogenatomen steht;

X für Wasserstoff, Fluor, Chlor, Brom und Alkyl oder Alkoxy mit jeweils 1 bis 4 Kohlenstoffatomen steht;

n für 1 steht und

Y für substituiertes Phenyl steht, wobei als Substituenten infrage kommen: Halogen oder Alkyl mit 1 bis 4 Kohlenstoffatomen,

sowie deren pflanzenverträgliche Säureadditions-Salze und Metallsalz-Komplexe.

2. Verfahren zur Herstellung von 2-Imidazolyl-2-phenyl-1,3-dioxolanen der Formel

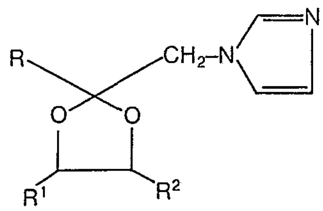

(I)

R für die Gruppierungen

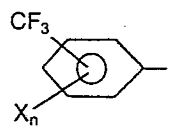 und 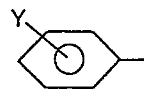 steht;

$R^1$ für Wasserstoff oder Alkyl mit 1 bis 4 Kohlenstoffatomen steht;

$R^2$ für Wasserstoff, Alkyl mit 1 bis 4 Kohlenstoffatomen oder Halogenalkyl mit 1 bis 2 Kohlenstoffatomen und 1 bis 5 gleichen oder verschiedenen Halogenatomen steht;

X für Wasserstoff, Fluor, Chlor, Brom und Alkyl oder Alkoxy mit jeweils 1 bis 4 Kohlenstoffatomen steht;

n für 1 steht und

Y für substituiertes Phenyl steht, wobei als Substituenten infrage kommen: Halogen oder Alkyl mit 1 bis 4 Kohlenstoffatomen,

sowie deren pflanzenverträglichen Säureadditions-Salzen und Metallsalz-Komplexen, dadurch gekennzeichnet, dass man 1,3-Dioxolan-Derivate der Formel

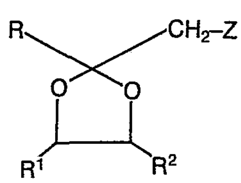

(II)

in welcher

R, $R^1$ und $R^2$ die oben angegebene Bedeutung haben und

Z für Halogen, sowie die Gruppierung $-O-SO_2-R^3$ steht, wobei

$R^3$ für Methyl oder p-Methylphenyl steht, mit Imidazolen der Formel

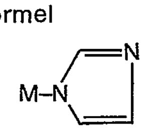

(III)

in welcher

M für Wasserstoff oder ein Alkalimetall steht, in Gegenwart eines Verdünnungsmittels und gegebenenfalls in Gegenwart eines Säurebindemittels umsetzt, und gegebenenfalls anschliessend an die so erhaltenen Verbindungen der Formel (I) eine Säure oder ein Metallsalz addiert.

3. Fungizide Mittel, gekennzeichnet durch einen Gehalt an mindestens einem 2-Imidazolylmethyl-2-phenyl-1,3-dioxolan der Formel (I) bzw. einem pflanzenverträglichen Säureadditions-Salz oder Metallsalz-Komplex von einem 2-Imidazolylmethyl-2-phenyl-1,3-dioxolan der Formel (I).

4. Verfahren zur Bekämpfung von Pilzen, dadurch gekennzeichnet, dass man 2-Imidazolylmethyl-2-phenyl-1,3-dioxolane der Formel (I) bzw. deren pflanzenverträgliche Säureadditions-Salze oder Metallsalz-Komplexe auf Pilze und/oder deren Lebensraum ausbringt.

5. Verwendung von 2-Imidazolylmethyl-2-phenyl-1,3-dioxolanen der Formel (I) bzw. von deren pflanzenverträglichen Säureadditions-Salzen und Metallsalz-Komplexen zur Bekämpfung von Pilzen.

6. Verfahren zur Herstellung von fungiziden Mitteln, dadurch gekennzeichnet, dass man 2-Imidazolylmethyl-2-phenyl-1,3-dioxolane der Formel (I) bzw. deren pflanzenverträgliche Säureadditions-Salze oder Metallsalz-Komplexe mit Streckmitteln und/oder oberflächenaktiven Stoffen vermischt.

7. 1,3-Dioxolan-Derivate der Formel

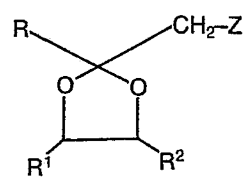

(II)

in welcher

R für die Gruppierungen

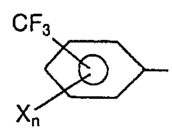 und 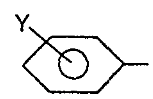 steht,

$R^1$ für Wasserstoff oder Alkyl mit 1 bis 4 Kohlenstoffatomen steht;

$R^2$ für Wasserstoff, Alkyl mit 1 bis 4 Kohlenstoffatomen oder Halogenalkyl mit 1 bis 2 Kohlenstoffatomen und 1 bis 5 gleichen oder verschiedenen Halogenatomen steht;

X für Wasserstoff, Fluor, Chlor, Brom und Alkyl oder Alkoxy mit jeweils 1 bis 4 Kohlenstoffatomen steht;

n für 1 steht und

Y für substituiertes Phenyl steht, wobei als Substituenten infrage kommen: Halogen oder Alkyl mit 1 bis 4 Kohlenstoffatomen,

und

Z für Halogen oder die Gruppe $-O-SO_2-R^3$ steht, wobei

$R^3$ für Methyl oder Tolyl steht.

8. Verfahren zur Herstellung von 1,3-Dioxolan-Derivaten der Formel

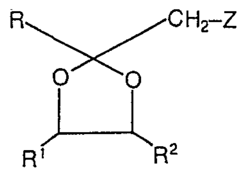

(II)

in welcher

R für die Gruppierungen

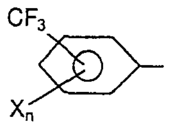 und 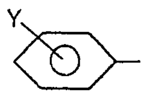 steht,

$R^1$ für Wasserstoff oder Alkyl mit 1 bis 4 Kohlenstoffatomen steht;

$R^2$ für Wasserstoff, Alkyl mit 1 bis 4 Kohlenstoffatomen oder Halogenalkyl mit 1 bis 2 Kohlenstoffatomen und 1 bis 5 gleichen oder verschiedenen Halogenatomen steht;

X für Wasserstoff, Fluor, Chlor, Brom und Alkyl oder Alkoxy mit jeweils 1 bis 4 Kohlenstoffatomen steht;

n für 1 steht und

Y für substituiertes Phenyl steht, wobei als Substituenten infrage kommen: Halogen oder Alkyl mit 1 bis 4 Kohlenstoffatomen,

und

Z für Halogen oder die Gruppe $-O-SO_2-R^3$ steht, wobei

$R^3$ für Methyl oder Tolyl steht,

dadurch gekennzeichnet, dass man Keto-Derivate der Formel

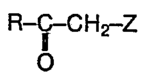

(IV)

in welcher

R und Z die oben angegebene Bedeutung haben,

mit Diolen der Formel

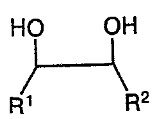

(V)

in welcher

$R^1$ und $R^2$ die oben angegebene Bedeutung haben,

in Gegenwart eines inerten organischen Lösungsmittels und in Gegenwart eines Katalysators umsetzt.

**Claims**

1. 2-Imidazolylmethyl-2-phenyl-1,3-dioxolanes of the formula

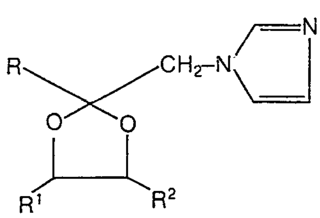

(I)

in which

R represents the groupings

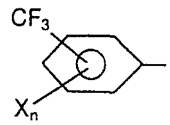 and 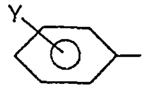

$R^1$ represents hydrogen or alkyl with 1 to 4 carbon atoms;

$R^2$ represents hydrogen, alkyl with 1 to 4 carbon atoms or halogenoalkyl with 1 to 2 carbon atoms and 1 to 5 identical or different halogen atoms;

X represents hydrogen, fluorine, chlorine, bromine and alkyl or alkoxy with in each case 1 to 4 carbon atoms;

n represents 1 and

Y represents substituted phenyl, possible substituents being: halogen or alkyl with 1 to 4 carbon atoms,

and their plant-tolerated acid addition salts and metal salt complexes.

2. Process for the preparation of 2-imidazolyl-2-phenyl-1,3-dioxolanes of the formula

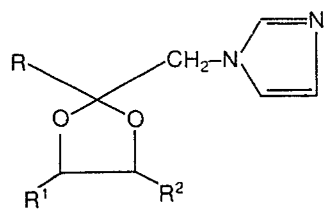

(I)

R represents the groupings

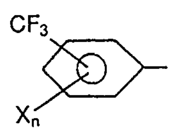 and 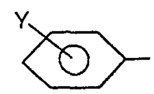

$R^1$ represents hydrogen or alkyl with 1 to 4 carbon atoms;

$R^2$ represents hydrogen, alkyl with 1 to 4 carbon atoms or halogenoalkyl with 1 to 2 carbon atoms and 1 to 5 identical or different halogen atoms;

X represents hydrogen, fluorine, chlorine, bromine and alkyl or alkoxy with in each case 1 to 4 carbon atoms;

n represents 1 and

Y represents substituted phenyl, possible substituents being: halogen or alkyl with 1 to 4 carbon atoms,

and their plant-tolerated acid addition salts and metal salt complexes, characterised in that 1,3-dioxolane derivatives of the formula

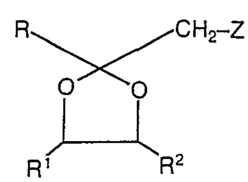

(II)

in which

R, $R^1$ and $R^2$ have the meaning given above and

Z represents halogen, and the grouping $-O-SO_2-R^3$,

wherein

$R^3$ represents methyl or p-methylphenyl, are reacted with imidazoles of the formula

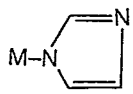

(III)

in which

M represents hydrogen or an alkali metal, in the presence of a diluent and, if appropriate, in the presence of an acid-binding agent, and, if desired, an acid or a metal salt is then added on to the compounds of the formula (I) thus obtained.

3. Fungicidal agents, characterised in that they contain at least one 2-imidazolylmethyl-2-phenyl-1,3-dioxolane of the formula (I) or a plant-tolerated acid addition salt or metal salt complex of a 2-imidazolylmethyl-2-phenyl-1,3-dioxolane of the formula (I).

4. Method of combating fungi, characterised in that 2-imidazolylmethyl-2-phenyl-1,3-dioxolanes of the formula (I) or their plant-tolerated acid addition salts or metal salt complexes are applied to fungi and/or their environment.

5. Use of 2-imidazolylmethyl-2-phenyl-1,3-dioxolanes of the formula (I) or of their plant-tolerated acid addition salts and metal salt complexes for combating fungi.

6. Process for the preparation of fungicidal agents, characterised in that 2-imidazolylmethyl-2-phenyl-1,3-dioxolanes of the formula (I) or their plant-tolerated acid addition salts or metal salt complexes are mixed with extenders and/or surface-active substances.

7. 1,3-Dioxolane derivatives of the formula

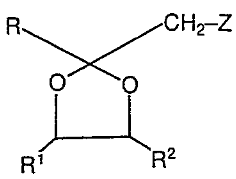

(II)

in which

R represents the groupings

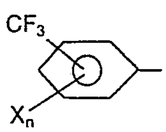

and

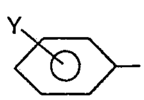

$R^1$ represents hydrogen or alkyl with 1 to 4 carbon atoms;

$R^2$ represents hydrogen, alkyl with 1 to 4 carbon atoms or halogenoalkyl with 1 to 2 carbon atoms and 1 to 5 identical or different halogen atoms;

X represents hydrogen, fluorine, chlorine, bromine and alkyl or alkoxy with in each case 1 to 4 carbon atoms;

n represents 1 and

Y represents substituted phenyl, possible substituents being: halogen or alkyl with 1 to 4 carbon atoms, and

Z represents halogen or the group $-O-SO_2-R^3$, wherein

$R^3$ represents methyl or tolyl.

8. Process for the preparation of 1,3-dioxolane derivatives of the formula

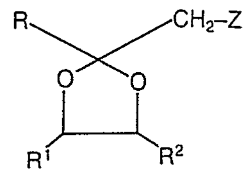

(II)

in which

R represents the groupings

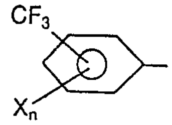

and

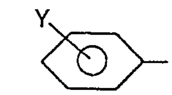

$R^1$ represents hydrogen or alkyl with 1 to 4 carbon atoms;

$R^2$ represents hydrogen, alkyl with 1 to 4 carbon atoms or halogenoalkyl with 1 to 2 carbon atoms and 1 to 5 identical or different halogen atoms;

X represents hydrogen, fluorine, chlorine, bromine and alkyl or alkoxy with in each case 1 to 4 carbon atoms;

n represents 1 and

Y represents substituted phenyl, possible substituents being: halogen or alkyl with 1 to 4 carbon atoms, and

Z represents halogen or the group $-O-SO_2R^3$, wherein

$R^3$ represents methyl or tolyl,

characterised in that keto derivatives of the formula

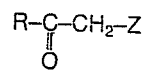

(IV)

in which

R and Z have the abovementioned meaning, are reacted with diols of the formula

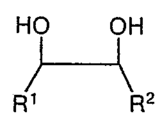

(V)

in which

$R^1$ and $R^2$ have the abovementioned meaning, in the presence of an inert organic solvent and in the presence of a catalyst.

**Revendications**

1. 2-imidazolylméthyl-2-phényl-1,3-dioxolannes de formule

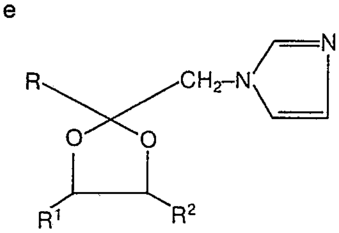

(I)

dans laquelle

R représente les groupements

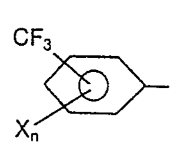

et

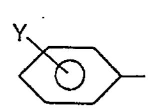

$R^1$ désigne l'hydrogène ou un groupe alkyle ayant 1 à 4 atomes de carbone;

$R^2$ est l'hydrogène, un groupe alkyle ayant 1 à 4 atomes de carbone ou un groupe halogénalkyle ayant 1 ou 2 atomes de carbone et 1 à 5 atomes d'halogènes identiques ou différents;

X représente l'hydrogène, le fluor, le chlore, le brome et des groupes alkyle ou alkoxy ayant chacun 1 à 4 atomes de carbone;

n a la valeur 1 et

Y est un groupe phényle substitué, et on considère alors comme substituants: un halogène ou un groupe alkyle ayant 1 à 4 atomes de carbone, ainsi que leurs sels d'addition d'acides et leurs complexes de sels métalliques compatibles avec les plantes.

2. Procédé de production de 2-imidazolyl-2-phényl-1,3-dioxolannes de formule

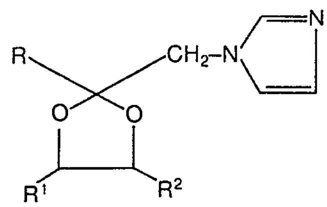 (I)

dans laquelle

R représente les groupements

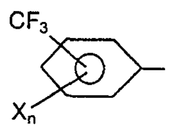 et 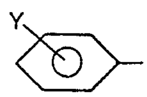

$R^1$ est l'hydrogène ou un groupe alkyle ayant 1 à 4 atomes de carbone;

$R^2$ désigne l'hydrogène, un groupe alkyle ayant 1 à 4 atomes de carbone ou un groupe halogénalkyle ayant 1 ou 2 atomes de carbone et 1 à 5 atomes d'halogènes identiques ou différents;

X est l'hydrogène, le fluor, le chlore, le brome et un groupe alkyle ou alkoxy ayant dans chaque cas 1 à 4 atomes de carbone;

n a la valeur 1 et

Y est un groupe phényle substitué, et on considère alors comme substituants: un halogène ou un groupe alkyle ayant 1 à 4 atomes de carbone, ainsi que de leurs sels d'addition d'acides et de leurs complexes de sels métalliques compatibles avec les plantes, caractérisé en ce qu'on fait réagir des dérivés de 1,3-dioxolanne de formule

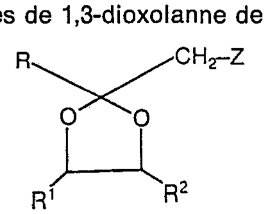 (II)

dans laquelle

R, $R^1$ et $R^2$ ont la définition indiquée ci-dessus et

Z désigne un halogène, ainsi que le groupement $-O-SO_2-R^3$, où $R^3$ est un groupe méthyle ou p-méthylphényle,

avec des imidazoles de formule

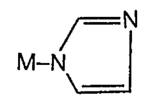 (III),

dans laquelle

M est l'hydrogène ou un métal alcalin,

en présence d'un diluant et, le cas échéant, en présence d'un accepteur d'acide, et on additionne ensuite, le cas échéant, un acide ou un sel métallique sur les composés de formule (I) ainsi obtenus.

3. Compositions fongicides, caractérisées par une teneur en au moins un 2-imidazolylméthyl-2-phényl-1,3-dioxolanne de formule (I) ou un sel d'addition d'acide ou un complexe de sel métallique compatible avec les plantes d'un 2-imidazolylméthyl-2-phényl-1,3-dioxolanne de formule (I).

4. Procédé pour combattre des champignons, caractérisé en ce qu'on applique des 2-imidazolylméthyl-2-phényl-1,3-dioxolannes de formule (I) ou leurs sels d'addition d'acides ou leurs complexes de sels métalliques compatibles avec les plantes à des champignons et/ou à leur milieu.

5. Utilisation de 2-imidazolylméthyl-2-phényl-1,3-dioxolannes de formule (I) ou de leurs sels d'addition d'acides et de leurs complexes de sels métalliques compatibles avec les plantes pour combattre des champignons.

6. Procédé de préparation de compositions fongicides, caractérisé en ce qu'on mélange des 2-imidazolylméthyl-2-phényl-1,3-dioxolannes de formule (I) ou leurs sels d'addition d'acides ou leurs complexes de sels métalliques compatibles avec les plantes avec des diluants et/ou des agents tensio-actifs.

7. Dérivés de 1,3-dioxolanne de formule

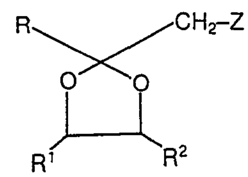 (II)

dans laquelle

R représente les groupements

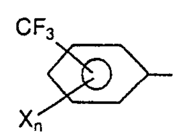 et 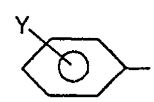

$R^1$ est l'hydrogène ou un groupe alkyle ayant 1 à 4 atomes de carbone;

$R^2$ est l'hydrogène, un groupe alkyle ayant 1 à 4 atomes de carbone ou un groupe halogénalkyle ayant 1 ou 2 atomes de carbone et 1 à 5 atomes d'halogènes identiques ou différents;

X désigne l'hydrogène, le fluor, le chlore, le brome et un groupe alkyle ou alkoxy ayant dans chaque cas 1 à 4 atomes de carbone;

n a la valeur 1 et

Y désigne un groupe phényle substitué, et on considère alors comme substituants un halogène

ou un groupe alkyle ayant 1 à 4 atomes de carbone,

et

Z est un halogène ou le groupe $-O-SO_2-R^3$, dans lequel

$R^3$ est un groupe méthyle ou tolyle.

8. Procédé de production de dérivés de 1,3-dioxolanne de formule

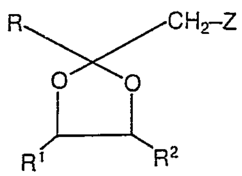

(II)

dans laquelle

R représente les groupements

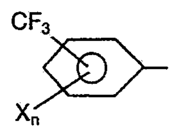

et

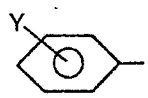

$R^1$ est l'hydrogène ou un groupe alkyle ayant 1 à 4 atomes de carbone;

$R^2$ est l'hydrogène, un groupe alkyle ayant 1 à 4 atomes de carbone ou un groupe halogénalkyle ayant 1 ou 2 atomes de carbone et 1 à 5 atomes d'halogènes identiques ou différents;

X désigne l'hydrogène, le fluor, le chlore, le brome et un groupe alkyle ou alkoxy ayant dans chaque cas 1 à 4 atomes de carbone;

n a la valeur 1 et

Y désigne un groupe phényle substitué, et on considère alors comme substituants un halogène ou un groupe alkyle ayant 1 à 4 atomes de carbone,

et

Z est un halogène ou un groupe $-O-SO_2-R^3$, dans lequel

$R^3$ est un groupe méthyle ou tolyle,

caractérisé en ce qu'on fait réagir des dérivés cétoniques de formule

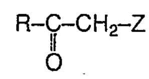

(IV)

dans laquelle

R et Z ont la définition indiquée ci-dessus, avec des diols de formule

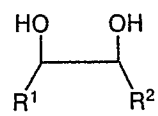

(V)

dans laquelle

$R^1$ et $R^2$ ont la définition indiquée ci-dessus, en présence d'un solvant organique inerte et en présence d'un catalyseur.